# EUROPÄISCHE PATENTANMELDUNG

(11) **EP 0 763 528 A2**
(43) Veröffentlichungstag der Anmeldung: **19.03.1997**
(21) Anmeldenummer: 96114458.1
(22) Anmeldetag: 10.09.1996
(51) Int. Cl.: C07D 203/02, B01J 35/02, B01J 29/03, B01J 29/04, B01J 29/82, B01J 29/89

(54) **Verfahren zur Herstellung von Aziridinen**

(30) Priorität: 12.09.1995 DE 19533662
(71) Anmelder: BASF AKTIENGESELLSCHAFT, 67056 Ludwigshafen (DE)
(72) Erfinder: Dingerdissen, Uwe, Dr., 64342 Seeheim-Jungenheim (DE); Lauth, Günter, Prof. Dr., 23909 Ratzenburg (DE); Trübenbach, Peter, Dr., 67059 Ludwigshafen (DE); Steuerle, Ulrich, Dr., 69124 Heidelberg (DE)
(74) Vertreter: Isenbruck, Günter, Dr.

(57) **Zusammenfassung**

Verfahren zur Herstellung von Aziridinen der allgemeinen Formel I 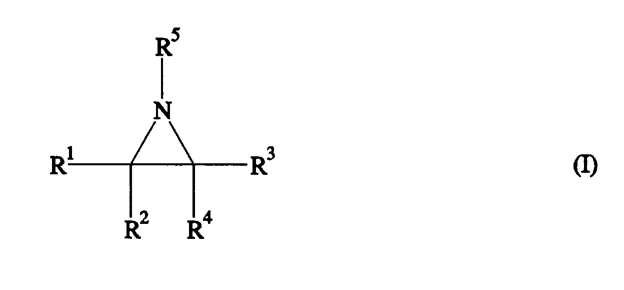 in der
- R¹,R²,R³,R⁴: Wasserstoff, C₁- bis C₈-Alkyl, C₃- bis C₈-Cycloalkyl oder Aryl und
- R⁵: Wasserstoff, C₁- bis C₈-Alkyl, C₃- bis C₈-Cycloalkyl, Aryl, Benzyl, C₁- bis C₈-Hydroxyalkyl oder C₁- bis C₈-Aminoalkyl bedeuten,
durch Umsetzung von Alkanolaminen der allgemeinen Formel II 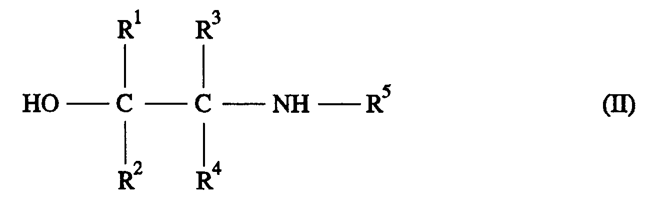 in der die Substituenten R¹, R², R³, R⁴ und R⁵ die die oben genannten Bedeutung haben, in der Gasphase bei Temperaturen von 200 bis 600°C und einem Druck von 0,001 bis 5bar an Heterogenkatalysatoren, indem man als Heterogenkatalysatoren vorgesinterte Formteile mit einheitlicher Porengröße im Makroporenbereich einsetzt.

## Beschreibung

Die vorliegende Erfindung betrifft ein Verfahren zur Herstellung von Aziridinen durch Umsetzung von Alkanolaminen an Sinterformteilen in der Gasphase.

Die Herstellung von Aziridinen, insbesondere Ethylenimin, durch Dehydratisierung von Alkanolaminen in der Gasphase an Molekularsieben aus Aluminiumsilikaten, Aluminiumphosphaten oder Silicium-Aluminiumphosphaten ist aus der WO-A-89/05797 bekannt.

Aus US-A-4 289 656, US-A-4 301 036, US-A-4 337 175, US-A-4 358 405, US-A-376 732 und US-A-4 477 591 sind Katalysatoren auf Basis Niob/Tantaloxid mit Zusatz von Erdalkalimetalloxiden und/oder Eisen/Chromoxiden zur katalytischen Gasphasendehydratisierung von Monoethanolamin zu Ethylenimin bekannt.

Weitere Katalysatoren zur intramolekularen Wasserabspaltung aus Alkanolaminen in der Gasphase sind Oxidmassen, die Silicium oder Phosphor als wesentliche Bestandteile enthalten. Katalysatoren dieser Art sind beispielsweise aus EP-A-227 461, EP-A-228 898 und EP-A-230 776 bekannt.

Die bekannten Katalysatoren haben den Nachteil, daß in unerwünschtem Maße Oligomere und Polymere des Aziridins entstehen. Ferner haben viele dieser Katalysatoren kurze Standzeiten.

Der vorliegenden Erfindung lag daher die Aufgabe zugrunde, den zuvor genannten Nachteilen abzuhelfen.

Demgemäß wurde ein neues und verbessertes Verfahren zur Herstellung von Aziridinen der allgemeinen Formel I 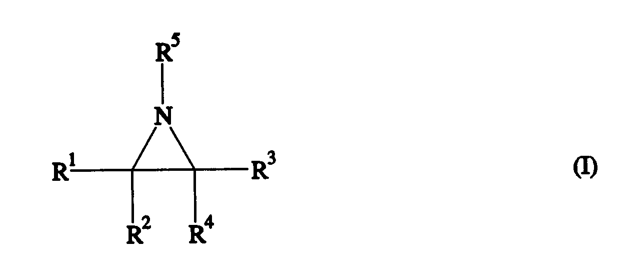 in der
- R¹,R²,R³,R⁴: Wasserstoff, C₁- bis C₈-Alkyl, C₃- bis C₈-Cycloalkyl oder Aryl und
- R⁵: Wasserstoff, C₁- bis C₈-Alkyl, C₃- bis C₈-Cycloalkyl, Aryl, Benzyl, C₁- bis C₈-Hydroxyalkyl oder C₁- bis C₈-Aminoalkyl bedeuten,
durch Umsetzung von Alkanolaminen der allgemeinen Formel II 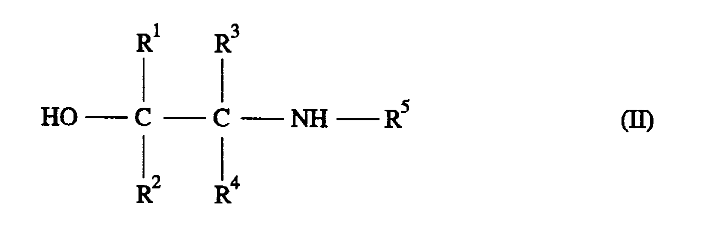 in der die Substituenten R¹, R², R³, R⁴ und R⁵ die die oben genannten Bedeutung haben, in der Gasphase bei Temperaturen von 200 bis 600°C und einem Druck von 0,001 bis 5 bar an Heterogenkatalysatoren gefunden, welches dadurch gekennzeichnet ist, daß man als Heterogenkatalysatoren vorgesinterte Formteile einsetzt.

Das erfindungsgemäße Verfahren läßt sich wie folgt durchführen:

Man kann die Alkanolamine II bei Temperaturen von 200 bis 600°C, bevorzugt 300 bis 550°C, besonders bevorzugt 350 bis 500°C und einem Druck von 0,001 bis 5 bar, bevorzugt 0,01 bis 2 bar, besonders bevorzugt 0,1 bis 1 bar in der Gasphase an einem vorgesinterten Formteil als Heterogenkatalysator diskontinuierlich oder bevorzugt kontinuierlich, stationär oder zirkulierend oder bevorzugt instationär in der Gasphasen-Fahrweise, bevorzugt in der Wirbelschicht umsetzen.

Als vorgesinterte Formteile eignen sich beispielsweise keramische Formkörper mit einheitlicher Porengröße im Makroporenbereich oder keramische Trägerteile mit einheitlicher Porengröße im Makroporenbereich und einer darauf fest anhaftenden katalytisch aktiven Schicht.

Bei der Herstellung von Aziridinen I kann die Dehydratisierung der Alkanolamine II in der stationären oder zirkulierenden Wirbelschicht durchgeführt werden. Bei der zirkulierenden Wirbelschicht (instationäre Fahrweise) wird der Katalysator kontinuierlich oder portionsweise ausgetauscht. Der aus dem Wirbelschichtreaktor ausgeschleusten Katalysator kann in einem anderen Reaktor bei Temperaturen von 300 bis 800°C, bevorzugt 400 bis 600°C und einem Druck von 0,01 bis 3 bar, bevorzugt 0,5 bis 1,5 bar, besonders bevorzugt Atmosphärendruck (Normaldruck), beispielsweise in einem Wirbelbett, in Gegenwart von Sauerstoff, beispielsweise in Form von Stickstoff und Luft-Gemischen, regeneriert werden. Der Reaktionsraum, in dem die Dehydratisierung erfolgt und der Regenerationsraum, in dem die aus dem Reaktor kommenden feinteiligen Katalysatoren mit Sauerstoff behandelt werden, müssen voneinander getrennt sein. Nach beendeter Regeneration und dem Entfernen des restlichen Sauerstoffs kann der feinteilige Katalysator wieder in den Wirbelschichtreaktor zurückgeführt werden. Die Geschwindigkeit, mit der die Katalysatormengen im Kreis geführt werden, wird vorzugsweise der Desaktivierungsgkinetik des Katalysators angepaßt. Die aus dem Wirbelschichtreaktor ausgeschleusten Katalysatormengen betragen pro 24 Stunden beispielsweise 0,1 bis 100 Gew.-%, vorzugsweise 0,5 bis 50 Gew.-% der Katalysatorgesamtmenge.

Die Isolierung der entstandenen Aziridine I aus dem Reaktionsgemisch kann beispielsweise mit Hilfe einer wäßrig-alkalischen Absorbtionsflüssigkeiten und nachgeschalteten Destillation erfolgen. Die wäßrig-alkalischen Absorptionsflüssigkeiten können mehrfach verwendet werden. Geeignete Flüssigkeiten dieser Art sind beispielsweise wäßrige NaOH oder KOH-Lösungen.

Wegen der hohen Reaktivität der Aziridine empfiehlt es sich, die heißen Reaktionsprodukte möglichst rasch abzukühlen. Man kann beispielsweise die aus dem Wirbelschichtreaktor austretenden heißen Reaktionsprodukte mit kondensierten Reaktionsprodukten, deren Temperatur höchstens 40°C beträgt, aus der Gasphase abscheiden. Die Temperatur der zum Kühlen verwendeten kondensierten Reaktionsprodukte liegt vorzugsweise in dem Bereich von -78°C bis +20°C. Nichtumgesetzte Alkanolamine II können aus dem Reaktionsgemisch isoliert und erneut zur Dehydratisierung eingesetzt werden.

Die erfindungsgemäßen Formkörper, welche als Katalysatoren oder Katalysatorträger eingesetzt werden können, zeichnen sich durch eine extrem monodispersen Porenverteilung aus, wobei die einheitliche Porengröße je nach Bedarf im Makroporenbereich eingestellt werden kann. Das Herstellverfahren der Formkörper ist universell auf eine Vielzahl von keramischen Pulvern und Pulvermischungen anwendbar. Als Geometrien der Formkörper sind alle Formen möglich, die über Granulierung, Walzen, Pressen, Strangpressen, Extrusion oder Spritzguß herstellbar sind wie beispielsweise Raschig Ringe, Wagenräderprofile, Fensterrahmenprofile, Honigwabenprofile, Sattelkörper, Sternringe, gelochte und/oder gerippte geometrische Körper wie Kugeln, Quader, Würfel, Kegel, Pyramiden, Prismen, Oktaeder, Zylinder, Pyramidenstümpfe und Kegelstümpfe, bevorzugt Ringe, Sattelkörper, Hohlkegel, besonders bevorzugt Ringe, Hohlkegel, die in der Regel ohne Nachbearbeitung hergestellt werden.

Die erfindungsgemäßen Formkörper lassen sich durch Verformen eines Gemisches aus einem keramischen Pulver und einem organischen Binder, Entfernen des Bindemittels durch Pyrolyse oder durch Behandlung mit einer gasförmigen Säure und Sintern zu einer Restporosität herstellen.

Im allgemeinen kann man die Formkörper wie folgt herstellen:

Nach Vermischen einer thermoplastischen Masse aus
A) 15 bis 70 Vol.-% eines keramischen Pulvers
B) 30 bis 85 Vol.-% eines thermoplastisch verformbaren Polymers und
C) 0 bis 15 Vol.-% eines Dispergierhilfsmittels
z.B. in einem Kneter, Extruder oder Scherwalzenextruder können diese Massen z.B. durch Verstrangen oder Spritzgießen bei Temperaturen von 160 bis 250°C, bevorzugt 170 bis 200°C und einem Druck von 500 bis 2000 bar, bevorzugt 180 bis 190 bar verformt werden.

Die auf Basis Polyacetal erhaltenen Grünkörper können einer gasförmigen, säurehaltigen Atmosphäre ausgesetzt werden. Die Temperatur der Bindemittelentfernung beträgt in der Regel 100 bis 160°C, bevorzugt 120 bis 140°C, und 0,1 bis 3 bar, bevorzugt Normaldruck (Atmosphärendruck), wobei man bevorzugt unter der Erweichungstemperatur des Binders bleibt.

Die Entfernung anderer Bindersysteme kann durch Pyrolyse bei 400 bis 800°C unter oxidierenden Bedingungen (Luft), Inertgas (N₂, Ar, He) oder reduzierenden Bedingungen (N₂/H₂, Ar/H₂) erfolgen.

Die Festigkeit und endgültige Porenverteilung der vorgesinterten Formteile wird in der Regel durch einen vom Pulver abhängigen Vorsinterprozeß, vorzugsweise bei 600 bis 1400°C, besonders bevorzugt bei 600 bis 800°C, unter oxidierenden Bedingungen (Luft), Inertgas (N₂, Ar, He) oder reduzierenden Bedingungen (N₂/H₂, Ar/H₂) eingestellt. Die mittlere Porengröße hängt in der Regel hauptsächlich von der Korngröße des Ausgangspulvers ab. Durch den Sinterprozeß wird die Porenverteilung in der Regel enger, während sich die Stabilität des porösen Formkörpers erhöht.

Die vorgesinterten Formteile, in Form von Katalysatoren oder Katalysatorträgern hat in der Regel eine einheitliche Porengröße im Bereich 50 nm bis 300 µm, wobei die Porengröße durch die Korngröße des eingesetzten Pulvers verändert werden kann.

Als keramische Pulver eignen sich beispielsweise SiC, WC, TiC, TaC, SiO₂, Al₂O₃, B₂O₃, TiO₂, Si₃N₄, BN, AlN, Alkali- oder Erdalkaliphosphate, -arsenate, -antimonate, -bismutate, -sulfate, -selenate, -tellurate oder deren Gemische, bevorzugt SiC, WC, TiC, TaC, SiO₂, Al₂O₃, B₂O₃, TiO₂ oder deren Gemische, besonders bevorzugt SiC, WC, TiC, TaC oder deren Gemische. Die Korngröße der verwendbaren Pulver geht von nanokristallinen Pulvern ab 0,005 µm bis 500 µm, vorzugsweise von 0,3 µm bis 100 µm, besonders bevorzugt von 0,5 bis 50 µm. Weiterhin können die Massen anorganische Fasern oder Whisker aus z.B. SiC zugesetzt werden.

Der organische Binder kann aus ein oder mehreren thermoplastischen Harzen, wie Polyacetal, Polyethylen, Polypropylen, Polystyrol, Polymethylmethacrylat und ein oder mehreren Weichmachern, wie Polyethylenglykol, Polypropylenglykol, Polybutandiolformal, Phthalsäureestern und Montanesterwachsen bestehen, wobei diese organischen Binder durch Pyrolyse entfernt werden können.

Im Falle eines Polyacetalbinders wird Polyoxymethylen, das vorteilhaft eine Molmasse von 10.000 bis 500.000 aufweist eingesetzt. Neben Homopolymerisaten von Formaldehyd oder Trioxan kommen auch Copolymerisate aus Trioxan mit z.B. cyclischen Ethern wie Ethylenoxid und 1,3-Dioxolan oder Formalen wie 1,3-Dioxepan, 1,3-Dioxan oder deren Mischungen oder homopolymeres Poly-1,3-dioxolan, Poly-1,3-dioxan, oder Poly-1,3-dioxepan in Betracht, wobei die Mengen der Copolymeren im allgemeinen bei 10 bis 30 Gew.-% der Polymeren liegen.

Ferner können Hilfsmittel mitverwendet werden wie Dispergatoren oder Schmiermittel, beispielsweise Polyethylenglykol, oder weitere thermoplastische Binder, wie Polyethylen, Polymethylmethacrylat oder Polyethylenoxid. Die Menge an Hilfsmittel liegt in der Regel zwischen 0,1 und 12 Gew.-% der Gesamtmasse.

Die erfindungsgemäßen Formkörper sind saure, neutrale und basische Katalysatoren oder Katalysatorträger, die bis zu Temperaturen, z.B. Al₂O₃: 1000°C, SiC: 1400°C, Fe/Ni: 800°C einsetzbar sind.

Zusätzlich kann man je nach Bedarf Trägermaterialien mit sehr hoher mechanischer oder chemischer Stabilität (Fe, SiC, Si₃N₄, Al₂O₃) einsetzen, die bisher mit den erfindungsgemäßen Porengrößenverteilungen nicht herstellbar waren. Auch hochgeglühte Pulver mit weitgehend inerter Oberfläche können mit dem erfindungsgemäßen thermoplastischen Binder gepreßt, stranggepreßt, extrudiert, spritzgegossen werden. Die Abriebfestigkeit kann durch eine Temperaturerhöhung beim Vorsintern erhöht werden. Da bei dem Calcinieren des Trägermaterials keine Aktivkomponente anwesend ist, können nach Tempern von über 550°C sehr hohe Festigkeiten des Trägermaterials erreicht werden.

Die erfindungsgemäß hergestellten Träger zeigen eine sehr gute Wasseraufnahme, so daß im Vergleich zu konventionellen Trägern mehr Aktivkomponente aufgebracht werden kann ohne Einbußen in der Härte hinnehmen zu müssen.

Die Formkörper, die zur Herstellung von Aziridinen eingesetzt werden, haben in der Regel auf den keramischen Trägern eine fest anhaftende katalytisch aktive Schicht, bevorzugt in Form einer Schale. Als Material für die katalytisch aktiven Schichten kommen sämtliche Verbindungen in Betracht, die für die intramolekulare Wasserabspaltung aus Alkanolaminen II z.B. aus SU-A-230166, JP-B-50/10593, WO-A-89/05797, EP-A-227461, EPA-228898 und EP-A-230776 bekannt sind. Bevorzugt werden dabei solche Alkali-, Erdalkali-, Lanthanidenverbindungen oder deren Gemische eingesetzt, die bei den Bedingungen der Aziridinsynthese stabil und insbesondere nicht flüchtig sind und keine flüchtigen Verbindungen bilden. Beispiele dafür sind Phosphate, Sulfate, Niobate, Wolframate, Vanadate, Manganate, Molybdate, Rhenate, Titanate und Salze von Heteropolysäuren. Die katalytisch aktiven Massen enthalten außerdem vorzugsweise Phosphor, Übergangsmetalle oder deren Gemische. Beispiele für solche katalytisch aktiven Massen sind mit Lithium dotiertes Wolframoxid; mit Natrium dotiertes Calciumwolframat; mit Cäsium oder Barium dotiertes Eisensulfat oder Nickelsulfat.

Als katalytisch aktive Massen eignen sich beispielsweise solche der allgemeinen Formel III

SiₐXₓY_{y}O_{b} (III),

in der Si Silicium bedeutet, X mindestens für ein Element aus der Gruppe der Alkali- und Erdalkalimetalle steht, Y ein Element aus der Gruppe B, Al, Ti, Zr, Sn, Zn und Ce ist und O Sauerstoff bedeutet und die Indices a, x, y und b die jeweiligen Atomverhältnisse der Elemente Si, X, Y und O angeben, wobei wenn a = 1, x = 0,005 bis 1, y = 0 bis 1 und b einen Wert hat, der durch a, x und y bestimmt wird.

Als katalytisch aktive Massen eignen sich weiterhin beispielsweise solche der allgemeinen Formel IV

XₐP_{b}X_{c}O_{d} (IV),

in der X mindestens ein Element bedeutet, ausgewählt aus Alkali- und Erdalkalimetallen. P Phosphor ist, Y mindestens ein Element ist, ausgewählt aus B, Al, Si, S, Sc, Ti, Cu, Y, Zr, Nb, Mo, Sn, Sb, La, Ce, Ta, W, Ti, Pb, Bi und Th, O Sauerstoff ist und die Indices a, b, c, und d die Atomverhältnisse der Elemente X, P, Y und O angeben und, wenn a = 1 ist, b = 0,01 bis 3 und C = 0 bis 100 ist und d ein Wert ist, durch a, b, und c und den Bindungszustand der einzelnen Elemente.

Als katalytisch aktive Massen eignen sich weiterhin beispielsweise solche der allgemeinen Formel V

XₐP_{b}Y_{c}O_{d} (V),

in der X mindestens ein Element ist, ausgewählt aus Elementen der Gruppe IIIA, Elementen der Gruppe IVA, Elementen der Gruppe VA, Übergangsmetallelementen der Gruppe I bis VIII, Lanthaniden- und Actinidenelementen des Periodensystems, P Phosphor ist, X mindestens ein Element ist, ausgewählt aus Alkali- und Erdalkalimetallen, O Sauerstoff bedeutet und die Indices a, b, c und d die Atomverhältnisse der Elemente X, P, Y und O angegeben und, wenn a = 1 ist, b = 0,01 bis 6 und c = 0 bis 3 ist und d ein Wert ist, bestimmt durch a, b, und c und den Bindungszustand der jeweiligen Elemente.

Weitere geeignete katalytisch aktive Massen sind die aus US-A-4337175, US-A 4,289,656, US-A 4,301,036, US-A 4,358,405, US-A 4,376,732 und US-A 4,477,591 bekannten Oxide von Tantal und Niob mit einem Erdalkalimetalloxid als Promotor.

Die Aufbringung der katalytisch aktiven Massen auf die keramischen Formkörper kann durch physikalische oder chemische Methoden geschehen, etwa durch Imprägnierung, Tränkung, physikalische oder chemische Abscheidung über die Dampfphase unter vermindertem Druck (physical oder chemical vapor deposition), z.B. Bedampfung oder Sputtern.

Die Substituenten R¹, R², R³, R⁴ und R⁵ in den Verbindungen I und II haben folgende Bedeutungen:
R¹,R²,R³,R⁴,R⁵
   - Wasserstoff,
   - C₁- bis C₈-Alkyl wie Methyl, Ethyl, n-Propyl, iso-Propyl, n-Butyl, iso--Butyl, sec.-Butyl, tert.-Butyl, n-Pentyl, iso-Pentyl, n-Hexyl, iso-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
   - C₃- bis C₈-Cycloalkyl wie Cyclopropyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
   - Aryl wie Phenyl, 1-Naphthyl und 2-Naphthyl, bevorzugt Phenyl,
R⁵
   - zusätzlich
   - Benzyl,
   - C₁- bis C₈-Hydroxyalkyl, bevorzugt C₁- bis C₄-Hydroxyalkyl, wie Hydroxymethyl, 1-Hydroxyethyl, 2-Hydroxyethyl, 1-Hydroxy-n-propyl, 2-Hydroxy-n-propyl und 3-Hydroxy-n-propyl, besonders bevorzugt C₁-bis C₂-Hydroxyalkyl wieHydroxymethyl, 1-Hydroxyethyl und 2-Hydroxyethyl,
   - C₁- bis C₈-Aminoalkyl, bevorzugt C₁- bis C₄-Aminoalkyl wie Aminomethyl, 1-Aminoethyl, 2-Aminoethyl, 1-Amino-n-propyl, 2-Amino-n-propyl und 3-Amino-n-propyl, besonders bevorzugt C₁- bis C₂-Aminoalkyl wie Aminomethyl, 1-Aminoethyl und 2-Aminoethyl.

### Beispiele

### Herstellung der Katalysatoren A bis D

### a) Verarbeiten von Pulver zu CIM-Material

Die in Tabelle 1 genannten Keramikpulver wurden mit einem Polyoxymethylencopolymer (POM/PBDF) aus Trioxan und 2,5 Gew.-% Butandiolformal mit einem durchschnittlichen Molekulargewicht von 150.000, sowie mit 20 Gew.-% Polybutandiolformal mit einem Molekulargewicht von 50.000 und 5 Gew.-% (bez. auf das eingesetzte Pulver) Polyethylenglykol mit einem Molekulargewicht von 800 als Hilfsmittel verknetet, zu Strängen mit einem Durchmesser von 4 mm extrudiert und in einem Entbinderungsofen mit 30 ml/h 100 Gew.-%iger Salpetersäure bei 130°C unter einem Stickstoffstrom von 300 l/h 10 Stunden säurekatalytisch entbindert. Anschließend erfolgte die aus Tabelle 1 ersichtliche Temperung (T₁).

### b₁) Aufbringen der Aktivmasse durch Besprühen

188,6 g des zuvor erhaltenen und entbinderten Formkörpers wurden zu Splitt (Teilchengröße 2 bis 3 mm) verarbeitet und bei einer Temperatur T₂ 5 Stunden vorcalciniert. Das Material wurde in einen beheizten Drehteller gegeben (Temperatur des Materials ca. 110°C) und mit einer Phosphorsäurelösung (6,8 g 85%-iger Phosphorsäure in 500 ml Wasser) besprüht. Die Lösung wurde zu einem feinen Nebel verdüst, der eine gleichmäßige Auftragung der Lösung auf die Splitt-Teilchen gewährleistete. Die Sprühdauer betrug ca. 4 Stunden. Das besprühte Material wurde bei 500°C 5 Stunden in einem Ofen zwischencalciniert, anschließend in den beheizten Drehteller mit einer Cäsium- und Barium-acetat-haltigen Lösung (4,6 g Bariumacetat und 6 g Cäsiumacetat in 500 ml Wasser) besprüht. Diese Behandlung wurde analog zur Phosphorsäurebehandlung durchgeführt. Das Material wurde anschließend bei 500°C 5 Stunden behandelt und bei der Temperatur T₃ endcalciniert.

### b₂) Aufbringen der Aktivmasse durch Imprägnierung

188,6 g des zuvor erhaltenen und entbinderten Formkörpers wurden zu Splitt (Teilchengröße 2 bis 3 mm) verarbeitet und bei einer Temperatur T₂ 5 Stunden vorcalciniert. Das Material wurde entsprechend seiner Wasseraufnahme mit einer wässrigen Phosphorsäurelösung (6,8 g 85%-iger Phosphorsäure in 500 ml Wasser) getränkt, bei 110°C 5 Stunden getrocknet und bei 500°C 5 Stunden zwischencalciniert. Anschließend wurde das Material entsprechend seiner Wasseraufnahme mit einer Cäsium- und Barium-acetat-haltigen Lösung (4,6 g Bariumacetat und 6 g Cäsiumacetat) getränkt. Das imprägnierte Material wurde bei 110°C 5 Stunden getrocknet, bei 500°C 5 Stunden calciniert und bei der Temperatur T₃ endcalciniert.

**Tabelle 1**

| Katalysator | Trägermaterial | Temperung (T₁)[°C] | Besprühen/Imprägnieren | Vorcalcinierung (T₂)[°C] | Endcalcinierung (T₃)[°C] |
|---|---|---|---|---|---|
| A | SiC (Stark, UF15) | 800 | Besprühen | 800 | 500 |
| B | ZrO2 (TOSOH, T2-3YS) | 800 | Besprühen | 800 | 500 |
| C | SiC (Starck, UF15) | 800 | Imprägnieren | 800 | 1000 |
| D | Si₃N4 (Starck, LC12) | 1000 | Imprägnieren | 1000 | 500 |

### Katalysator E

### Herstellung von Calciumphosphat

118,1 g Calciumnitrat-tetrahydrat wurden in 200 ml Wasser gelöst, auf 80°C erwärmt, eine Lösung aus 33 g Di-ammoniumphosphat in 100 ml Wasser gegeben, mit Ammoniak basisch gestellt und 30 Minuten gealtert. Nach Abkühlen, Filtration und Waschen wurde der Niederschlag bei 120°C getrocknet und 2 Stunden bei 600°C calciniert. Das erhaltene Pulver wurde gemäß der Katalysatorherstellung a) in einen erfindungsgemäßen Formkörper übergeführt.

### Vergleichsbeispiel I

Das analog Katalysator E erhaltene Pulver wurde mit 5 Gew.-% Methylcellulose und Wasser zu einer viskosen Masse angeteigt und mit einer Strangpresse zu Strängen mit einem Durchmesser von 3mm verarbeitet (Preßdruck ca. 70 bar). Die Stränge wurden 5 Stunden bei 110°C getrocknet und bei 500°C 5 Stunden calciniert.

### Katalysator F

### Herstellung von Bariumphosphat

63,1 g Bariumhydroxid-octahydrat wurden in 100 ml Wasser aufgeschlämmt, auf 90°C erwärmt, 18,5 g Diammoniumphosphat unter Rühren zugegeben, die Mischung eingeengt, bei 120°C getrocknet und bei 600°C 2 Stunden calciniert. Das erhaltene Pulver wurde gemäß der Katalysatorherstellung a) in einen erfindungsgemäßen Formkörper übergeführt.

### Vergleichsbeispiel II

Das analog KatalysatorF erhaltene Pulver wurde mit 5 Gew.-% Methylcellulose und Wasser zu einer viskosen Masse angeteigt und mit einer Strangpresse zu Strängen mit einem Durchmesser von 3 mm verarbeitet (Preßdruck ca. 70 bar). Die Stränge wurden 5 Stunden bei 110°C getrocknet und bei 500°C 5 Stunden calciniert.

### Katalysator G

### Natrium-haltiges Calciumphosphat

118,1 g Calciumnitrat-tetrahydrat wurden in 200 ml Wasser gelöst, auf 80^{o}C erwärmt, unter Rühren eine Lösung von 107,4 g Dinatriumhydrogenphosphat-dodecahydrat in 200 ml Wasser zugegeben, mit Ammoniak basisch gestellt und 60 Minuten gealtert. Nach Abkühlen, Filtration und Waschen wurde der Niederschlag bei 120°C getrocknet und 2 Stunden bei 500°C calciniert. Das erhaltene Pulver wurde gemäß der Katalysatorherstellung a) in einen erfindungsgemäßen Formkörper übergeführt.

### Vergleichsbeispiel III

Das analog Katalysator F erhaltene Pulver wurde mit 5 Gew.-% Methylcellulose und Wasser zu einer viskosen Masse angeteigt und mit einer Strangpresse zu Strängen mit einem Durchmesser von 3 mm verarbeitet (Preßdruck ca. 70 bar). Die Stränge wurden 5 Stunden bei 110°C getrocknet und bei 500°C 5 Stunden calciniert.

### Herstellung von Aziridin

In einem mit 20 bis 100 g Katalysator gefüllten kontinuierlich betriebenen, beheizbaren Linearreaktor (Innendurchmesser 30 mm, Länge 300 mm) wurden in Gasphasen-Fahrweise 120 l/h Stickstoff und 30 g/h Ethanolamin II (Wassergehalt bis zu 1 Gew.-%) zudosiert.

Die Austräge wurden gaschromatographisch getrennt nach Flüssig- und Gasphase untersucht. Die Flüssigphase wurde mit Natronlauge stabilisiert. Die Ergebnisse sind in den Tabellen 2 und 3 zusammengestellt.

**Tabelle 2**

| **Reaktionstemperatur 400°C** | | | | | | |
|---|---|---|---|---|---|---|
| Katalysator | Zusammensetzung | | | Umsatz [%] | Selektivität [%] | Ausbeute [%] |
| | Barium [Gew.-%] | Cäsium [Gew.-%] | Phosphor [Gew.-&] | | | |
| A | 1,3 | 2,1 | 1 | 81,2 | 9,1 | 7,4 |
| B | 1,3 | 2,1 | 1 | 68,4 | 6,8 | 4,7 |
| C | 1,35 | 2,3 | 0,9 | 57,7 | 56,3 | 32,5 |
| D | 1,3 | 2,1 | 1 | 84,7 | 13,8 | 11,7 |
| E | | | | 88,6 | 4,3 | 3,8 |
| F | 68,4 | 0 | 10,3 | 81,4 | 41,3 | 33,6 |
| G | | | | 95,6 | 11 | 10,5 |
| V-I | | | | 33,7 | 0 | 0 |
| V-II | 67 | 0 | 12,2 | 27,9 | 10,1 | 2,8 |
| V-III | | | | 45,4 | 4 | 1,8 |

**Tabelle 3**

| **Reaktionstemperatur 450°C** | | | | | | |
|---|---|---|---|---|---|---|
| Katalysator | Zusammensetzung | | | Umsatz [%] | Selektivität [%] | Ausbeute [%] |
| | Barium [Gew.-%] | Cäsium [Gew.-%] | Phosphor [Gew.-&] | | | |
| A | 1,3 | 2,1 | 1 | 69,1 | 7,3 | 5 |
| B | 1,3 | 2,1 | 1 | 35,5 | 3,8 | 1,3 |
| C | 1,35 | 2,3 | 0,9 | 51,7 | 44,4 | 23 |
| D | 1,3 | 2,1 | 1 | 52,2 | 5,9 | 3,1 |
| E | | | | 62,6 | 1,6 | 1 |
| F | 68,4 | 0 | 10,3 | 62,1 | 67,1 | 41,7 |
| G | | | | 78,7 | 54,8 | 43,1 |
| V-I | | | | 17,4 | 0 | 0 |
| V-II | 67 | 0 | 12,2 | 30,6 | 15,3 | 4,7 |
| V-III | | | | 32,9 | 2,2 | 0,7 |

## Patentansprüche

1. Verfahren zur Herstellung von Aziridinen der allgemeinen Formel I 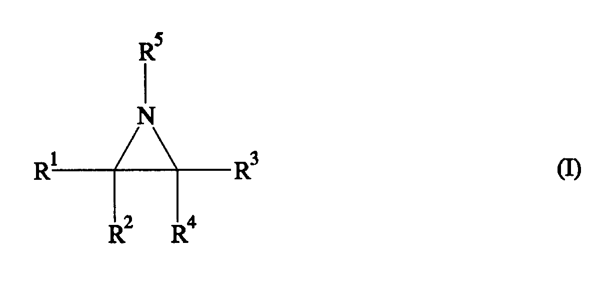 in der
R¹,R²,R³,R⁴ Wasserstoff, C₁- bis C₈-Alkyl, C₃- bis C₈-Cycloalkyl oder Aryl und
R⁵ Wasserstoff, C₁- bis C₈-Alkyl, C₃- bis C₈-Cycloalkyl, Aryl, Benzyl, C₁- bis C₈-Hydroxyalkyl oder C₁- bis C₈-Aminoalkyl bedeuten,
durch Umsetzung von Alkanolaminen der allgemeinen Formel II 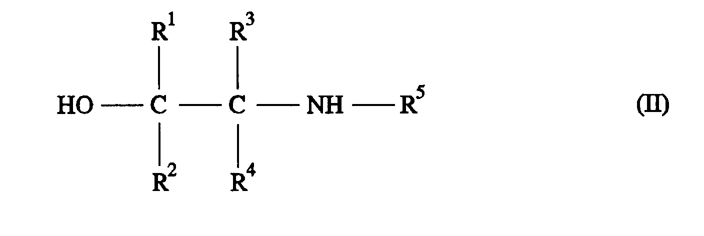 in der die Substituenten R¹, R², R³, R⁴ und R⁵ die die oben genannten Bedeutung haben, in der Gasphase bei Temperaturen von 200 bis 600°C und einem Druck von 0,001 bis 5 bar an Heterogenkatalysatoren, dadurch gekennzeichnet, daß man als Heterogenkatalysatoren vorgesinterte Formteile einsetzt.

2. Verfahren zur Herstellung von Aziridinen nach Anspruch 1, dadurch gekennzeichnet, daß man die Umsetzung in der Wirbelschicht bei einem Druck von 0,001 bis 2 bar durchführt.

3. Verfahren zur Herstellung von Aziridinen nach den Ansprüchen 1 bis 2, dadurch gekennzeichnet, daß man die Umsetzung unter einem Druck von 0,01 bis 1 bar durchführt.

4. Verfahren zur Herstellung von Aziridinen nach den Ansprüchen 1 bis 3, dadurch gekennzeichnet, daß man die Umsetzung in einer stationären oder einer zirkulierenden Wirbelschicht durchführt.

5. Verfahren zur Herstellung von Aziridinen nach den Ansprüchen 1 bis 4, dadurch gekennzeichnet, daß man vorgesinterte Formkörper mit einheitlicher Porengröße im Bereich 50 nm bis 300 µm einsetzt, hergestellt durch
a) Verformen eines Gemisches aus einem keramischen Pulver und einem organischen Binder zu einer thermoplastischen Masse durch Granulierung, Pressen, Walzen, Extrusion, Spritzguß,
b) Entfernen des Bindemittels durch Pyrolyse oder durch Behandlung mit einer gasförmigen Säure und
c) Sintern.

6. Verfahren zur Herstellung von Aziridinen nach den Ansprüchen 1 bis 5, dadurch gekennzeichnet, daß die Formkörper vor der Sinterung thermoplastischen Massen aus
A) 15 bis 70 Vol.-% eines keramischen Pulvers
B) 30 bis 85 Vol.-% eines thermoplastisch verformbaren Polymers und
C) 0 bis 15 Vol.-% eines Dispergierhilfsmittels
sind.

7. Verfahren zur Herstellung von Aziridinen nach Anspruch 6, dadurch gekennzeichnet, daß die keramischen Pulver A) in den Formkörpern oxidische, karbidische oder nitridische Pulver wie SiO₂, Al₂O₃, TiO₂, ZrO₂, B₂O₃, Y₂O₃, Si₃N₄, BN, AlN, TiN, ZrN, SiC, TiC, WC, B₄C oder deren Gemische sind.

8. Verfahren zur Herstellung von Aziridinen nach Anspruch 6, dadurch gekennzeichnet, daß die keramischen Pulver A) in den Formkörpern Alkali- oder Erdalkaliphosphate, -arsenate, -antimonate, -bismutate, -selenate, -tellurate oder deren Gemische sind.

9. Verfahren zur Herstellung von Aziridinen nach Anspruch 6, dadurch gekennzeichnet, daß die keramischen Pulver A) in den Formkörpern Wolframate, Niobate, Wolframatekieselsäuren oder deren Gemische sind.

10. Verfahren zur Herstellung von Aziridinen nach den Ansprüchen 6 bis 9, dadurch gekennzeichnet, daß die Formkörper eine fest anhaftende katalytisch aktive Schicht eines Elementes der Alkali-, Erdalkali-, Lanthanidengruppe des Periodischen Systems oder deren Gemische oder von Verbindungen von Hauptgruppenelementen der 3. bis 7. Hauptgruppe enthalten.
